# EUROPÄISCHE PATENTANMELDUNG

(11) **EP 1 537 848 A1**
(43) Veröffentlichungstag der Anmeldung: **08.06.2005**
(21) Anmeldenummer: 04105536.9
(22) Anmeldetag: 05.11.2004
(51) Int. Cl.: A61K 7/06

(54) **Haar- und/oder Kopfhautpflegemittel mit Licochalcon A**

(30) Priorität: 03.12.2003 DE 10356866
(71) Anmelder: Beiersdorf AG, 20245 Hamburg (DE)
(72) Erfinder: Kruse, Inge, 20146, Hamburg (DE); Mummert, Christopher, 29553, Bienenbüttel (DE); Kolbe, Ludger, 21255, Dohren (DE); Demitz, Michael, 22529, Hamburg (DE); Argembeaux, Horst, 21465, Wentorf (DE); Wallendszus, Ines, 22761, Hamburg (DE)

(57) **Zusammenfassung**

Kosmetische Haar- und/oder Kopfhautpflegemittel enthaltend Licochalcon A oder einen Extrakt aus Radix Glycrrhizae inflatae die als Haarkur, Haarspülung, Haarwasser oder als Kopfhauttonikum vorliegen.

## Beschreibung

Die vorliegende Erfindung betrifft kosmetische Haarpflegezubereitungen, Haar- und/oder Kopfhautpflegemittel mit einem Gehalt an Licochalcon A, insbesondere zur Verbesserung des Zustandes gereizter Kopfhaut, beispielsweise nach einer Shampoobehandlung.

Der ganze menschliche Körper mit Ausnahme der Lippen, der Handinnenflächen und der Fußsohlen ist behaart, zum Großteil allerdings mit kaum sichtbaren Wollhärchen. Wegen der vielen Nervenenden an der Haarwurzel reagieren Haare empfindlich auf äußere Einflüsse wie Wind oder Berührung und sind daher ein nicht zu unterschätzender Bestandteil des Tastsinns. Die wichtigste Funktion des menschlichen Kopfhaares dürfte allerdings heute darin bestehen, das Aussehen des Menschen in charakteristischer Weise mitzugestalten. Ähnlich wie die Haut erfüllt es eine soziale Funktion, da es über sein Erscheinungsbild erheblich zu zwischenmenschlichen Beziehungen und zum Selbstwertgefühl des Individuums beiträgt.

Das Haar besteht aus dem frei aus der Haut herausragenden Haarschaft - dem keratinisierten (toten) Teil, der das eigentlich sichtbare Haar darstellt - und der in der Haut steckenden Haarwurzel - dem lebenden Teil, in dem das sichtbare Haar ständig neu gebildet wird. Der Haarschaft seinerseits ist aus drei Schichten aufgebaut: einem zentralen Teil - dem sogenannten Haarmark (Medulla), welches allerdings beim Menschen zurückgebildet ist und oft gänzlich fehlt - ferner dem Mark (Cortex) und der äußeren, bis zu zehn Lagen starken Schuppenschicht (Cuticula), die das ganze Haar umhüllt.

Das menschliche Haar ist, sofern keine krankhaften Veränderungen vorliegen, in seinem frisch nachgewachsenen Zustand praktisch nicht zu verbessern. Der in der Nähe der Kopf-haut befindliche Teil eines Haares weist dementsprechend eine nahezu geschlossene Schuppenschicht auf. Insbesondere die Schuppenschicht als Außenhülle des Haares, aber auch der innere Bereich unterhalb der Cuticula sind besonderer Beanspruchung durch Umwelteinflüsse ausgesetzt.

Wesentliche Einflüsse für den Qualitätsverlust eines Haares während seiner Alterung sind der Einfluss des Sonnenlichts, mechanische Belastungen durch intensives Kämmen oder Bürsten, aber auch Haarbehandlungen, wie Haarfärbungen und insbesondere Blondierungen sowie Haarverformungen, beispielsweise Dauerwellverfahren. Besonders oxidative Belastungen führen demnach häufig zu einer Schädigung des Haares.

Sowohl UV-A- als auch UV-B-Strahlung haben einen schädigenden Einfluss auf das Haar, der sich beispielsweise darin äußert, dass bestimmte Aminosäuren wie Cystin und Methionin abgebaut oder Schwefel-Schwefel-Bindungen des Keratins gespalten werden, was im schlimmsten Fall eine Zerstörung des Haars zur Folge haben kann. Weiterhin stellen Haar und Kopfhaut Teile des Körpers dar, die aufgrund ihrer Position beim Aufenthalt im Freien einer erheblichen Menge an UV-Strahlung ausgesetzt sind.

Ein Ziel der Haarpflege ist es, den Naturzustand des frisch nachgewachsenen Haares über einen möglichst langen Zeitraum zu erhalten und im Fall eines Verlusts wieder herzustellen. Seidiger Glanz, geringe Porosität und ein angenehmes, glattes Gefühl gelten als Merkmale für natürliches, gesundes Haar.

Seit Ende des vergangenen Jahrhunderts werden Produkte zur Haarpflege gezielt entwickelt. Dies führte zu einer Vielzahl von Präparaten sowohl für die allgemeine Haarpflege als auch zur Behebung von Anomalien des Haares und der Kopfhaut. Im allgemeinen werden heutzutage Haarpflegekosmetika verwendet, welche entweder dazu bestimmt sind, nach dem Einwirken aus dem Haar wieder ausgespült zu werden, oder welche auf dem Haar verbleiben sollen. Letztere können so formuliert werden, daß sie nicht nur der Pflege des einzelnen Haars dienen, sondern auch das Aussehen einer Frisur insgesamt verbessern. Solcherart gepflegtes Haar zeichnet sich durch einen angenehmen Griff, natürlichen Glanz, vermehrte Fülle, Geschmeidigkeit und somit gute Frisierbarkeit und Festigkeit und somit gutem Frisurensitz aus.

Produkte die ausschließlich der Pflege des Haares dienen, werden allgemein als Haarkonditioniermittel oder Conditioner bezeichnet. Diese können nach einer mehr oder weniger langen Verweilzeit auf dem Haar ausgespült werden (Rinse-off Produkte, z.B. Spülungen, Haar-Kuren) oder sie verbleiben nach der Anwendung auf dem Haar (Leave-on Produkte). Die Produkte können verschiedene Konsistenzen haben, so dass sie ganz unterschiedlich appliziert werden können. Es können Emulsionen oder Gele sein oder dünnflüssige Lösungen, die z.B. über Sprühapplikationen aufgebracht werden, oder Schäume, die z.B. durch geeignete Druckgaspackungen oder spezielle Schaumpumpen bei der Applikation erzeugt werden. Cremige, trübe und klar transparente Produkte sind im Markt zu finden.

Je nach Verwendungszweck findet man ganz unterschiedliche Wirkstoffe oder Kombinationen von Wirkstoffen in solchen Conditionern. Manche, die eher dem Schutz des Haares dienen, wie Antioxidantien oder UV-Filter, andere die das Haar geschmeidig machen wie z.B. kationische Tenside. Eine immer größere Bedeutung bekommen polymere Wirkstoffe, die je nach Art, Molgewicht und Ladung ganz unterschiedliche Eigenschaften haben. Im Vordergrund steht dabei jedoch eindeutig eine Verbesserung der Oberflächenbeschaffenheit des Haares, jedoch nicht eine Verbesserung der Haut, die von der Haarbehandlung stets mit betroffen wird. Diese Haut, insbesondere die Epidermis, ist als Barriereorgan des menschlichen Organismus in besonderem Maße äußeren Einwirkungen unterworfen. Derartige Einwirkungen können einen Reiz auf die Haut ausüben, die bei Menschen mit sensibler, empfindlicher oder verletzlicher Haut zu Rötung, Spannung, Brennen und / oder Juckreiz führen können. Zu solchen Einwirkungen zählen UV-Bestrahlung, mechanische Belastungen, Kontakt mit irritierenden Stoffen. Zu den Hautarealen, die diesen Einwirkungen weitgehend ungeschützt ausgesetzt sind und empfindlich reagieren gehört insbesondere die Kopfhaut.

Neben der Sonnenbestrahlung der die Kopfhaut je nach Haartracht mehr oder weniger ungeschützt ausgesetzt ist, mechanischer Belastungen durch Kämmen, Bürsten, Schneiden inc. Rasieren, ist die Kopfhaut auch dem Kontakt mit irritierenden Stoffen ausgesetzt. Neben Chemikalien, die beim Haarfärben oder Dauerwellen eingesetzt werden, sind hier insbesondere die Tenside zu erwähnen, die beim häufig täglichen Haarwaschen zum Einsatz gelangen.

Nach dem Haarwaschen wird das Haar und/oder die Kopfhaut häufig weiteren Behandlungen ausgesetzt die das Haar pflegen und die Kopfhaut stimulieren sollen. Bei Produkten, die primär die Kopfhaut stimulieren handelt es sich meist um Haarwässer oder um Haar Tonika. Solche Haarwässer sind i.d.R. wässrig-alkoholische Lösungen, die ggf. weitere Wirkstoffe enthalten.

Sowohl die Conditioner als auch die Haarwässer sind gut hautverträgliche Produkte, es ist jedoch nicht zu erwarten, dass durch entsprechende Anwendung bereits vorgeschädigte Kopfhaut in ihrem Zustand verbessert wird. Gelegentlich können bei gereizter Kopfhaut eher Verstärkungen einiger Symptome beobachtet werden. Licochalcone A ist Bestandteil des Auszugs aus Radix Glycrrhizae inflatae und zeichnet sich durch folgende Struktur aus:

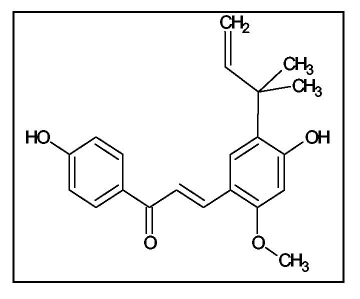

Die Pflanzenart Glycrrhizae inflatae gehört wie das in Europa offiizinelle Süßholz (Glycrrhiza glabra) der Gattung Glycrrhiza an, die zur Pflanzenfamilie der Fabaceae (Erbsengewächse) gehört.

Es hat sich nun überraschend und für den Fachmann nicht vorhersehbar herausgestellt, daß kosmetische Haar- und/oder Kopfhautpflegemittel enthaltend Licochalcon A oder den Extrakt aus Radix Glycrrhizae inflatae die als Haarkur, Haarspülung, Haarwasser oder als Kopfhauttonikum vorliegen, den Nachteilen des Standes der Technik abhelfen. Durch solche Zubereitungen wird die Kopfhaut, die mit dem Mittel bei der Anwendung in Kontakt kommt, besonders gepflegt indem der Zustand gereizter Kopfhaut verbessert wird. Die Ursache gereizter Kopfhaut kann UV-Bestrahlung, mechanische Beanspruchung und/oder Chemikalienkontakt sein. Insbesondere der Chemikalienkontakt und hierbei ist ganz besonders der häufige Kontakt mit Reinigungstensiden zu nennen ist ein häufig auftretender Stressfaktor für die Kopfhaut, dessen Symptome, wie Rötungen, Brennen, Jucken, durch die erfindungsgemäßen Produkte deutlich gemindert oder beseitigt werden, ohne die Pflegeeigenschaften und die Qualität und Verteilbarkeit des Mittels in unguter Weise zu beeinflussen. Ein derart verbesserter Zustand ist besonders bei tensidgeschädigter Kopfhaut zu beobachten. Der Extrakt aus Radix Glycrrhizae inflatae kann wässrig oder alkoholisch sein. Der alkoholische Extrakt ist aufgrund seiner Wirksamkeit besonders bevorzugt. Erhältlich sind solche Extrakte beispielsweise unter den Bezeichnungen "Polyol Soluble Licorice Extract P-U" oder "Aqua Licorice Extract P-U" der Fa. Maruzen Pharmaceuticals. Der erstgenannte Extrakt stellt einen alkoholischen Extrakt dar, der in Pulverform vorliegt. "Aqua Licorice Extract P-U" enthält noch zusätzliche ethoxilierte und/oder propoxilierte Rohstoffe. Dabei ist es von Vorteil, wenn diese ethoxilierten und/oder propoxilierten Rohstoffe, die vom Hersteller den Extrakten aus verschiedenen Gründen zugesetzt werden, in der Zubereitung nicht enthalten sind, weil deren mögliches Sensibilisierungspotential dann nicht in Erscheinung treten kann. Insbesondere Abwesenheit von PPG-6 (Nikkol PEN) ist von großem Vorteil. Ganz besonders vorteilhaft ist es, von einem alkoholischen Extrakt auszugehen, wie er unter der Bezeichnung Polyol Soluble Licorice Extract PU (INCI-Bezeichnung Glycyrrhiza Inflata) von der Firma Maruzen zu erhalten ist. Der Extrakt aus *Radix Glycyrrhizae inflatae* enthält einen Anteil von ca. 25 % Licochalcon A.

Es ist bevorzugt, wenn der Gehalt an Licochalcon A oder Extrakt aus Radix Glycrrhizae inflatae 0,001 bis 10 Gew.%, besonders bevorzugt 0,05 bis 5 Gew.%, ganz besonders bevorzugt 0,01 bis 2 Gew.-% beträgt, jeweils bezogen auf die Gesamtformulierung. Weiter bevorzugt enthalten solche Pflegemittel zusätzlich weitere kosmetische oder dermatologische Hilfs-, Zusatz- und/oder Wirkstoffe. Solche Ppflegemittel können in Form einer Haarkur oder Haarspülung bevorzugt zusätzlich quartäre Ammoniumverbindungen, in Form eines Haarwassers oder Kopfhauttonikums zusätzlich Ethanol oder Isopropanol enthalten.

Die Erfindung umfasst auch die Verwendung der beschriebenen Pflegemittel zur Behandlung tensidgeschädigter Kopfhaut, ein Produkt umfassend eines der beschriebenen Pflegemittel enthalten in einem beschrifteten Packmittel, wobei die Beschriftung auf die Verwendung zur Behandlung tensidgeschädigter Kopfhaut hinweist sowie ein nicht therapeutisches Verfahren zur Behandlung von tensidgeschädigter Kopfhaut dadurch gekennzeichnet, dass ein erfindungsgemäßes Pflegemittel topisch angewandt wird.
Erfindungsgemäße kosmetische und dermatologische Zubereitungen können in verschiedenen Formen vorliegen. So können sie z.B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsionen, beispielsweise vom Typ Wasser-in-Öl-in-Wasser (W/O/W), ein Gel, einen festen Stift, eine Salbe oder auch ein Aerosol darstellen.

Die Haarpflegemittel sind topische Zubereitungen. Diese können wie üblich zusammengesetzt sein und zur Behandlung und der Pflege der Kopfhaut und/oder der Haare oder als Lichtschutzpräparat dienen. Zur Anwendung werden die erfindungsgemäßen Zubereitungen in der für Kosmetika und Haarpflegemittel üblichen Weise auf die Kopfhaut und die Haare in ausreichender Menge aufgebracht.

Vorteilhaft können Zubereitungen im Sinne der vorliegenden Erfindung als Haarkur oder Haarspülung vorliegen.

Die Mittel gemäß der Erfindung können beispielsweise als aus Aerosolbehältern, Quetschflaschen oder durch eine Pump-, Sprüh- oder Schaumvorrichtung versprühbare Präparate vorliegen, jedoch auch in Form eines aus normalen Flaschen und Behältern auftragbaren Mittels.

Als Treibmittel für aus Aerosolbehältern versprühbare kosmetische oder dermatologische Zubereitungen im Sinne der vorliegenden Erfindung, sind die üblichen bekannten leichtflüchtigen, verflüssigten Treibmittel, beispielsweise Dimethylether, Kohlenwasserstoffe (Propan, Butan, Isobutan) geeignet, die allein oder in Mischung miteinander eingesetzt werden können. Auch Druckluft, Stickstoff, Stickstoffdioxid oder Kohlendioxid oder Gemische aus diesen Substanzen sind vorteilhaft zu verwenden.

Natürlich weiß der Fachmann, dass es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Vorteilhaft enthalten erfindungsgemäße Zubereitungen neben einem wirksamen Gehalt an erfindungsgemäßen Wirkstoffen ferner übliche Wirk-, Inhalts-, Zusatz- und/oder Hilfsstoffe.

Als Haarpflegemittel wird eine Vielzahl von Produkten bezeichnet, deren wichtigste Vertreter Vorbehandlungsmittel, Haarwässer und Haarkonditioniermittel sind.

Grundstoffe für Haarpflegemittel sind z.B. Fettalkohole, Wachse, Paraffine, Vaseline, Paraffinöl und Lösemittel.

Fettalkohole sind z.B. gerad- oder verzweigtkettige aliphatische einwertige Alkohole mit 6 - 22 C-Atomen im Molekül. In der Kosmetik werden vorzugsweise geradkettige Fettalkohole mit einer Kettenlänge von 12 - 18 C-Atomen verwendet. Diese Fettalkohole sind weiche, farblose Massen, praktisch ungiftig und gut hautverträglich. Fettalkohole werden bevorzugt zur Herstellung von Haarkuren und Frisiercremes verwendet, wobei dem Cetylalkohol und dem Stearylalkohol besondere Bedeutung zukommt.

Wachse sind Fettsäureester, die in tierischen und pflanzlichen Produkten vorkommen, aber auch synthetisch hergestellt werden können. Das wohl bekannteste natürlich vorkommende Wachs ist das Bienenwachs, das als Hauptbestandteile Cerin und Myricin enthält. Wachs ist jedoch ein Oberbegriff für eine Reihe natürlich oder künstlich gewonnener Stoffe, die in der Regel halbfeste, weiße, geruchlose und in Wasser unlösliche Massen darstellen.

Paraffine im kosmetischen Sinn sind weiße, geruchlose Massen aus geradkettigen hochmolekularen Kohlenwasserstoffen. Wegen ihrer den der Wachse vergleichbaren Eigenschaften werden sie auch oft als Erdölwachse bezeichnet.

Vaseline ist ein Gemisch von verzweigtkettigen Paraffinen mit geringem Anteil an zyklischen Paraffinen. Es ist eine weiche, transparente und in Wasser unlösliche Masse mit geringem Eigengeruch, die bei der Aufbereitung des Erdöls anfällt.

Paraffinöl ist ein Gemisch gesättigter flüssiger Kohlenwasserstoffe. Es ist unlöslich in Wasser, aber mischbar mit Fettalkoholen und Wachsen. Es wird als Zusatz in Haarpflegemitteln zur Konsistenzregulierung verwendet.

Lösemittel spielen in der Kosmetik eine erhebliche Rolle. Von der großen Anzahl Lösemittel, die zur Verfügung stehen, hat Ethanol die größte Bedeutung. Es wird zur Herstellung von Haarwässern verwendet, in denen es wegen seiner desinfizierenden Eigenschaften gleichzeitig die Funktion eines Wirkstoffes erfüllt. Gelegentlich wird auch Isopropanol für diese Zwecke eingesetzt.

Hilfstoffe können verwendet werden, um bestimmte Eigenschaften der Haarpflegemittel, z.B. Konsistenz, Temperatur- und Lichtstabilität, Aussehen und Geruch, zu verbessern sowie deren Herstellung zu erleichtern. Zugesetzt werden z.B. nach Bedarf:
- Emulgatoren, um die Grenzflächenspannung zwischen zwei an sich nicht mischbaren Phasen so weit herabzusetzen, daß deren feine Vermischung möglich wird,
- Verdickungsmittel, um die Stabilität von Emulsionen zu erhöhen und deren Viskosität einzustellen,
- UV-Absorber, um die Lichtstabilität der in Haarpflegemitteln enthaltenen Farbstoffe und anderer lichtempfindlicher Komponenten zu verbessern. Daneben dienen sie dem Schutz des Haares vor Lichteinflüssen.
- Konservierungsmittel, um mikrobieller Zersetzung vorzubeugen.
- Antioxidantien, um Geruchsveränderungen, die durch Oxidationsvorgänge hervorgerufen werden können, zu verhindern.
- Farbstoffe, um Haarpflegemitteln ein ansprechendes Aussehen zu verleihen.
- Parfümöle, um Haarpflegemitteln einen angenehmen Duft zu verleihen und Nebengerüche der Rohstoffe zu überdecken.

Die Menge der Grundstoffe beträgt beispielsweise 85 bis 99,999 Gew.-%, vorzugsweise 90 bis 99,99 Gew.-%, jeweils bezogen auf das Gesamtgewicht der Zubereitung.

Quaternäre Ammonium-Verbindungen sind eine wichtige Gruppe der speziellen Wirkstoffe, die zur Herstellung von Haarpflegemitteln verwendet werden. Haarpflegemittel, insbesondere Haarkuren, erhalten wesentliche Eigenschaften wie Verbesserung von Kämmbarkeit sowie Griff und Verhinderung statischer Aufladung der Haare vornehmlich durch den Einsatz quaternärer Ammonium-Verbindungen. Die Eigenschaften quaternärer Ammonium-Verbindungen werden durch die kationische Gruppe einerseits und durch die Art der lipophilen Reste dieser Gruppe andererseits bestimmt. Geeignet sind z.B. die Verbindungen, in denen ein bis zwei Reste längerkettige Alkyl-Gruppen, wie Lauryl-, Cetyl- oder Stearyl-Gruppen, und die verbliebenen Reste Methyl-Gruppen sind. Produkte dieses Typs werden vorzugsweise als Chloride, Bromide und Methosulfate eingesetzt.

Geeignet sind auch polymere quaternäre Ammonium-Verbindungen, Makromoleküle, deren wesentliches Merkmal das Vorhandensein mehrerer quaternärer AmmoniumGruppen im Molekül ist. Dadurch wird ihre Haftfähigkeit am Haar deutlich erhöht.

Besonders vorteilhaft zu verwendende kationische Tenside sind
1. Alkylamine
2. Alkylimidazole
3. Ethoxylierte Amine und
4. Quaternäre Tenside.
5. Esterquats

Quaternäre Tenside erhalten mindestens ein N-Atom, das mit 4 Alkyl- oder Arylgruppen kovalent verbunden ist. Dies führt, unabhängig vom pH-Wert, zu einer positiven Ladung. Vorteilhaft sind Alkylbetain, Alkylamidopropylbetain und Alkyl-amidopropylhydroxysultain. Die erfindungsgemäß verwendeten kationischen Tenside können ferner bevorzugt gewählt werden aus der Gruppe der quaternären Ammoniumverbindungen, insbesondere Benzyltrialkylammoniumchlorid, ferner Alkyltrialkylammoniumsalze, beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

Monomere oder polymere quaternäre Ammonium-Verbindungen werden vielfach in Haarspülungen und Haarkuren z.B. in Konzentrationen von 0,5 - 5 Gew.-% eingesetzt. Dazu gehören Cetrimoniumchlorid wie es unter der Bezeichnung Dehyquart A von der Gesellschaft Cognis angeboten wird oder Distearoylethyl Hydroxyethylmonium Methosulfate wie es unter der Bezeichnung Dehyquart F 75 von der Gesellschaft Cognis angeboten wird.

Liegen die kosmetischen oder dermatologischen Zubereitungen in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wird, so handelt es sich dabei z.B. um Emulsionen, die gegebenenfalls oberflächenaktive Substanzen enthalten, deren Konzentration zwischen 0,1 und 10 Gew.-%, vorzugsweise zwischen 0,2 und 5 Gew.-%, liegen kann.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine Emulsion dar und enthält die erfindungsgemäßen Kombinationen. Es ist allerdings gegebenenfalls vorteilhaft, wenn die erfindungsgemäße Lotion in Form einer Mikroemulsion oder einer wässrigen oder wässrig-alkoholischen Lösung vorliegt.

Erfindungsgemäß können kosmetische Zubereitungen zur Behandlung und Pflege der Haare als Gele vorliegen, die organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z.B. Aluminiumsilikate wie beispielsweise Bentonite, oder ein Gemisch aus Polyethylenglykol und Polyethylenglycolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Die vorstehenden Prozentangaben beziehen sich auf das Gesamtgewicht der Zubereitungen.

Die erfindungsgemäßen Zubereitungen können kosmetische Hilfsstoffe enthalten, wie sie üblicherweise in solchen Zubereitungen verwendet werden, z.B. Konservierungsmittel, Parfüme, Substanzen zum Verhindern des Schäumens, Schaumstabilisatoren, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, rückfettende Agentien, Fette, Öle, Wachse, Alkohole, Polyole und deren toxikologisch verträglichen Ether und Ester, verzweigte und/oder unverzweigte Kohlenwasserstoffe, weitere Antioxidantien, Stabilisatoren, pH-Wert-Regulatoren, Konsistenzgeber, Bakterizide, Desodorantien, antimikrobielle Stoffe, Antistatika, UV-Absorber, Komplexierungs- und Sequestrierungsagentien, Perlglanzagentien, Polymere, Elektrolyte, organische Lösungsmittel, Silikonderivate, Pflanzenextrakte, Vitamine und/oder andere Wirkstoffe oder andere übliche Bestandteile einer kosmetischen oder dermatologischen Formulierung. Auch Lösungsvermittler, z.B. zur Einarbeitung hydrophober Komponenten wie z.B. von Parfümzubereitungen können enthalten sein.

Die Gesamtmenge der Hilfsstoffe beträgt beispielsweise 0,001 bis 15 Gew.-%, vorzugsweise 0,01 bis 10 Gew.-%, jeweils bezogen auf das Gesamtgewicht der Zubereitung.

Die Menge der Verdickungsmittel beträgt beispielsweise 0,05 bis 5,0 Gew.-%, vorzugsweise 0,1 bis 3,0 Gew.-%, insbesondere 0,15 bis 2,0 Gew.-%, jeweils bezogen auf das Gesamtgewicht der Zubereitung.

Der Wassergehalt der Zubereitungen beträgt beispielsweise 60 bis 95 Gew.-%, vorzugsweise 75 bis 95 Gew.-%, insbesondere 80 bis 90 Gew.-%, jeweils bezogen auf das Gesamtgewicht der Zubereitung.

Erfindungsgemäß können zusätzlich als Antioxidantien alle für kosmetische und/oder dermatologische Anwendungen geeigneten oder gebräuchlichen Antioxidantien verwendet werden.

Die Gesamtmenge der Antioxidantien beträgt beispielsweise 0,000.001 bis 2 Gew.-%, vorzugsweise 0,001 bis 1 Gew.-%, jeweils bezogen auf das Gesamtgewicht der Zubereitung.

Vorteilhaft werden weitere Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α -Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und des sen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Vorteilhaft können erfindungsgemäße Zubereitungen außerdem Substanzen enthalten, die UV-Strahlung im UV-B-Bereich absorbieren, wobei die Gesamtmenge der Filtersubstanzen z.B. 0,001 Gew.-% bis 30 Gew.-%, vorzugsweise 0,05 bis 10 Gew.-%, insbesondere 0,1 bis 1,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut, insbesondere die Kopfhaut dienen.

Enthalten die erfindungsgemäßen Emulsionen UV-B-Filtersubstanzen, können diese vorteilhaft wasserlöslich sein. Vorteilhafte wasserlösliche UV-B-Filter sind z.B.:
- Salze der 2-Phenylbenzimidazol-5-sulfonsäure wie ihr Natrium-, Kalium- oder ihr Triethanolammonium-Salz, sowie die 2-Phenylbenzimidazol-5-sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Es kann auch von Vorteil sein, erfindungsgemäße Zubereitungen mit UV-A-Filtern zu versetzen, die bisher üblicherweise in kosmetischen Zubereitungen enthalten sind. Es können die für die UV-B-Kombination verwendeten Mengen eingesetzt werden.

Die Herstellung der erfindungsgemäßen Zubereitungen kann in der üblichen Weise durch Mischen der einzelnen Bestandteile erfolgen. Die Wirkstoffe der erfindungsgemäßen Kombinationen oder auch die vorgemischten Bestandteile der erfindungsgemäßen Kombinationen können im Mischvorgang zugegeben werden.

Der pH-Wert der Zubereitungen kann in bekannter Weise durch Zugabe von Säuren oder Basen eingestellt werden, vorzugsweise durch Zugabe von Puffergemischen, z.B. auf Basis von Citronensäure/Citrat oder Phosphorsäure Phosphat-Puffergemischen. Vorzugsweise liegt der pH-Wert unter 10, z.B. im Bereich von 2-7, insbesondere im Bereich von 3-5.

Alle Mengenangaben, Anteile und Prozentanteile sind, soweit nicht anders angegeben, auf das Gewicht und die Gesamtmenge bzw. auf das Gesamtgewicht der Zubereitungen oder der jeweiligen Mischung bezogen.

Die folgenden Beispiele verdeutlichen die Erfindung.
Die Mengenangaben in den Beispielen sind Gew.-% bezogen auf das Gesamtgewicht der jeweiligen Zubereitung.

### Beispiele

| **Beispiel Haarkuren:** | | | |
|---|---|---|---|
| | **1** | **2** | **3** |
| Hydroxypropylmethylcellulose | 0,5% | 0,5% | 0,5% |
| Cetrimoniumbromid | 1,0 | - | 0,8 |
| Behentrimoniumchlorid | - | 0,7 | 0,3 |
| Glycerin | 3,0 | 3,0 | 3,0 |
| Cetearylalkohol | 2,5 | 2,5 | 2,5 |
| Glycerylstearat | 2,0 | 2,0 | 2,0 |
| Polyquaternium-10 | 0,1 | - | - |
| Guar Hydroxypropyl Trimonium Chlorid | - | 0,2 | - |
| Licorice Extract P-U* | 0,2 | 0,1 | 0,25 |
| Konservierungsmittel, Parfüm, pH-Einstellung und Lösungsvermittler | q.s. | q.s. | q.s. |
| Wasser | ad 100 | ad 100 | ad 100 |

| **Beispiel Haarspülungen:** | | | |
|---|---|---|---|
| | **1** | **2** | **3** |
| Cetrimoniumchlorid | 1,0 | 0,5 | 0,5 |
| Behentrimoniumchlorid | - | 0,2% | 0,3 |
| Glycerin | 3,0 | 3,0 | 3,0 |
| Hydroxyethylcellulose | 0,2 | 0,2 | 0,2 |
| Polyquaternium-10 | 0,1 | - | - |
| Guar Hydroxypropyl Trimonium Chlorid | - | 0,2 | - |
| Licorice Extract P-U* | 0,05 | 0,2 | 0,15 |
| Konservierungsmittel, Parfüm, pH-Einstellung und Lösungsvermittler | q.s. | q.s. | q.s. |
| Wasser | ad 100 | ad 100 | ad 100 |

| **Beispiel Spray-Conditioner:** | | | | |
|---|---|---|---|---|
| | **1** | **2** | **3** | **4** |
| Cetrimoniumchlorid | 0,2 | 0,1 | 0,8 | 0,5 |
| Behentrimoniumchlorid | - | 0,2 | 0,3 | - |
| Benzophenone-4 | 0,05 | 0,03 | - | - |
| PVP/VA Copolymer | - | 0,7 | - | - |
| Polyquaternium-10 | 0,1 | - | - | - |
| Polyquaternium-4 | 0,2 | - | - | - |
| Propylene Glycol | - | - | - | 3,0 |
| Polyquaternium-11 | - | - | 0,2 | - |
| Panthenol | 0,1 | - | 0,2 | 0,1 |
| Glyceryllsostearate | - | - | - | 0,4 |
| Isoceteth-20 | - | - | - | 0,8 |
| Dicaprylyl Carbonate | - | - | - | 0,5 |
| Licorice Extract P-U* | 0,025 | 0,05 | 0,1 | 0,07 |
| Konservierungsmittel, Parfüm, pH-Einstellung und Lösungsvermittler | q.s. | q.s. | q.s. | q.s. |
| Wasser | ad 100 | ad 100 | ad 100 | ad 100 |

| **Beispiel Leave-on Conditioner:** | | | | |
|---|---|---|---|---|
| | **1** | **2** | **3** | **4** |
| Cetylalcohol | 1,5 | 1,8 | 2,0 | - |
| Cetrimoniumchlorid | 0,3 | 0,1 | 0,5 | 0,5 |
| Behentrimoniumchlorid | - | 0,2 | - | - |
| Benzophenone-4 | 0,05 | 0,03 | - | 0,1 |
| PVP/VA Copolymer | 0,4 | - | - | - |
| Polyquaternium-37 | - | - | - | 1,0 ' |
| Polyquaternium-4 | - | - | - | 0,2 |
| Polyquaternium-10 | - | - | 0,5 | - |
| Panthenol | 0,1 | - | 0,2 | 0,1 |
| Hydroxyethylcellulose | - | - | - | 0,3 |
| Acrylates/C10-30 Alkyl Acrylates Crosspolymer | 0,5 | 0,3 | 0,2 | - |
| C12-13 Alkyl Lactate | 2,0 | 1,0 | 1,5 | 1,0 |
| Laureth-4 | - | - | - | 0,5 |
| Aluminium Starch Octenylsuccinate | - | - | - | 1,0 |
| Dicaprylyl Carbonate | - | - | - | 1,0 |
| Licorice Extract P-U* | 0,03 | 0,1 | 0,05 | 0,08 |
| Konservierungsmittel, Parfüm, pH-Einstellung und Lösungsvermittler | q.s. | q.s. | q.s. | q.s. |
| Wasser | ad 100 | ad 100 | ad 100 | ad 100 |

| **Beispiel Haarwässer:** | | | | |
|---|---|---|---|---|
| | **1** | **2** | **3** | **4** |
| Ethanol | 30,0 | 50,0 | - | - |
| Isopropanol | - | - | 40,0 | 30,0 |
| Panthenol | 0,2 | 0,1 | 0,2 | 0,2 |
| Menthol | 0,1 | - | 0,05 | 0,05 |
| Tocopheryl Acetate | 0,2 | 0,2 | - | 0,1 |
| C12-13 Alkyl Lactate | 0,2 | 0,1 | 0,2 | - |
| Licorice Extract P-U* | 0,04 | 0,03 | 0,1 | 0,05 |
| Parfüm, pH-Einstellung und Lösungsvermittler | q.s. | q.s. | q.s. | q.s. |
| Wasser | ad 100 | ad 100 | ad 100 | ad 100 |

| | | | | |
|---|---|---|---|---|
| *Polyol Soluble Licorice Extract P-U (Glycyrrhiza Inflata) der Fa. Maruzen Pharmaceuticals | | | | |

## Patentansprüche

1. Kosmetische Haar- und/oder Kopfhautpflegemittel enthaltend Licochalcon A oder einen Extrakt aus Radix Glycrrhizae inflatae die als Haarkur, Haarspülung, Haarwasser oder als Kopfhauttonikum vorliegen.

2. Pflegemittel nach einem der vorangehenden Ansprüche **dadurch gekennzeichnet, daß** der Gehalt an Licochalcon A oder der Extrakt aus Radix Glycrrhizae inflatae 0,001 bis 5 Gew.%, besonders bevorzugt 0,005 bis 2 Gew.%, ganz besonders bevorzugt 0,01 bis 0,1 Gew.-% bezogen auf das Gesamtgewicht der Zubereitung, beträgt.

3. Pflegemittel nach einem der vorangehenden Ansprüche, **dadurch gekennzeichnet, daß** zusätzlich weitere kosmetische oder dermatologische Hilfs-, Zusatzund/oder Wirkstoffe enthalten sind.

4. Haarkur oder Haarspülung nach einem der vorangehenden Ansprüche **dadurch gekennzeichnet, dass** sie zusätzlich quartäre Ammoniumverbindungen enthalten.

5. Haarwasser oder Kopfhauttonikum nach einem der vorangehenden Ansprüche **dadurch gekennzeichnet, dass** sie zusätzlich Ethanol oder Isopropanol enthalten.

6. Verwendung von Pflegemittel nach einem der vorangehenden Ansprüche zur Behandlung, insbesondere zur Verbesserung des Zustandes vorgeschädigter Kopfhaut, bevorzugt tensidgeschädigter Kopfhaut.

7. Produkt umfassend ein Pflegemittel nach einem der vorangehenden Ansprüche enthalten in einem beschrifteten Packmittel, wobei die Beschriftung auf die Verwendung nach Anspruch 6 hinweist.

8. Nicht therapeutisches Verfahren zur Behandlung von tensidgeschädigter Kopfhaut **dadurch gekennzeichnet, dass** ein Pflegemittel nach einem der vorangehenden Ansprüche topisch angewandt wird.
